# EUROPÄISCHE PATENTSCHRIFT

(11) **EP 4 271 286 B1**
(45) Veröffentlichungstag und Bekanntmachung des Hinweises auf die Patenterteilung: **24.07.2024**
(21) Anmeldenummer: 23701459.2
(22) Anmeldetag: 20.01.2023
(51) Int. Cl.: A61B 17/16

(54) **SCHAFT EINES ODER FÜR EIN MEDIZINISCHES HANDINSTRUMENT**
SHAFT OF OR FOR A MANUAL MEDICAL INSTRUMENT
ARBRE D'INSTRUMENT MÉDICAL MANUEL OU POUR UN TEL INSTRUMENT

(30) Priorität: 27.01.2022 DE 102022101885
(43) Veröffentlichungstag der Anmeldung: 08.11.2023
(73) Patentinhaber: Aesculap AG, 78532 Tuttlingen (DE)
(72) Erfinder: BÜRK, André, 78056 Villingen-Schwenningen (DE); GRIMM, Christian, 78532 Tuttlingen (DE); KILLINGER, Lilian, 71139 Ehningen (DE)
(74) Vertreter: Winter, Brandl - Partnerschaft mbB
(86) Internationale Anmeldenummer: PCT/EP2023/051398
(87) Internationale Veröffentlichungsnummer: WO 2023/144036

(56) Entgegenhaltungen:
- US-A1- 2017 333 052
- US-A1- 2021 153 913

## Beschreibung

### Technisches Gebiet

Die vorliegende Offenbarung betrifft einen Schaft eines oder für ein medizinisches Handinstrument und ein medizinisches Handinstrument.

### Hintergrund der Offenbarung

Abwinkelbare Schäfte für medizinische Handinstrumente sind aus dem Stand der Technik bekannt. Medizinischen Handinstrumente der vorliegenden Gattung sind beispielsweise Bohrer oder Fräser, die bei minimal-invasiven Operationen eingesetzt werden. Diese haben in der Regel einen Handgriff, an dem ein Instrumentenschaft montiert oder montierbar ist, wie er Gegenstand der vorliegenden Offenbarung ist. Im Schaft ist ein Antriebsstrang oder Antriebszug aufgenommen, der ein Drehmoment von einer Antriebsquelle/Motoreinheit, bevorzugt innerhalb des Handgriffs auf einen Effektor (Fräser, Bohrer) am distalen Endabschnitt des Schafts überträgt.

Bei chirurgischen Eingriffen spielt der Bauraum der Instrumente und eine gute Handhabbarkeit eine große Rolle. So sollen die Instrumentenschäfte insbesondere im Bereich der distalen Effektoren auf möglichst kleinem Raum aktiv (über einen Betätigungsmechanismus gewollt ausgeführt) abwinkelbar sein.

Ein beispielhafter Schaft weist hierfür einen distalen und einen proximalen Schaftabschnitt auf. Der distale Schaftabschnitt ist relativ zu dem proximalen Schaftabschnitt um seine Längsachse drehbar. Da eine jeweils stirnseitige Auflagefläche der beiden Schaftabschnitte jeweils bezüglich der Schaftlängsachse in einem Winkel, unterschiedlich zu 90° in zueinander gleicher Weise angewinkelt/schräggestellt sind, wird der distale Schaftabschnitt durch dessen Drehung relativ zu dem proximalen Schaftabschnitt abgewinkelt oder gerade ausgerichtet. So können der Schaft des medizinischen Handinstruments und damit der Effektor während des Einsatzes des Handinstruments bezüglich des Handgriffs abgewinkelt werden.

Um den distalen Schaftabschnitt zu drehen und dabei/dadurch gleichzeitig relativ zum proximalen Schaftabschnitt abzuwinkeln, muss eine Drehbewegung oder Rotation ausgehend vom proximalen auf den distalen Schaftabschnitt übertragen werden. Hierfür ist ein Getriebezug innerhalb des Schafts vorgesehen, der im proximalen Schaftabschnitt drehgelagert und am distalen Schafsabschnitt drehfest montiert ist. An der Stelle, in der der Schaft abgewinkelt wird, muss demzufolge über einen geeigneten Schaftdrehmechanismus innerhalb des Getriebezugs ein Drehmoment auf den distalen Schaftabschnitt übertragen werden, wobei der Schaftdrehmechanismus aber auch eine Biegung/Abwinklung ermöglichen muss. Deshalb erfordert der Schaftdrehmechanismus an dieser Stelle des Schaftes ein Bauteil oder eine Konstruktion mit einer notwendigen Torsionssteifigkeit bei gleichzeitiger Biege-/Abwinkelbarkeit. Ferner soll der Schaft einen möglichst kleinen Außendurchmesser aufweisen und weiterhin ein möglichst positionsgenaues Verdrehen und damit Abwinkeln des distalen Schaftabschnitts ermöglichen. Auch muss innerhalb des Schafts ausreichend Platz für den Antriebsstrang des Effektors selbst erhalten bleiben.

### Stand der Technik

Ein interner Stand der Technik der Anmelderin schlägt für das vorstehend genannte Bauteil oder Konstruktion ein flexibles Federblech mit einer Kugel an einem Ende des Federblechs vor. Das flexible Federblech verbindet die proximalen und distalen Schaftabschnitte, die sich gegeneinander verdrehen sollen. Die Kugel ist derart in einer Nut an dem einen Schaftabschnitt eingebracht, dass sie axial beweglich ist, aber Rotationen überträgt. Das Ende des Federblechs, das der Kugel gegenüberliegt, ist fest mit dem anderen Schaftabschnitt verbunden. Wenn nun der eine Schaftabschnitt gedreht wird, überträgt das Federblech die Rotation. Das Federblech kann sich aber auch gleichzeitig verbiegen, um zusammen mit dem distalen Schaftabschnitt bezüglich des proximalen Schaftabschnitts abgewinkelt/ abgeknickt zu werden.

Es hat sich aber herausgestellt, dass diese Konstruktion nicht das nötige Drehmoment auf den distalen Schaftabschnitt übertragen kann. Es wurden daher weitere Ausführungsformen vorgeschlagen, die alle aus verschiedenen Gründen nicht als Schaftdrehmechanismus zwischen distalem und proximalem Schaftabschnitt geeignet sind. So wurde auch ein flexibler Silikonschlauch angedacht, der aber das nötige Drehmoment vom Getriebezug innerhalb des proximalen Schaftabschnitts auf den distalen Schaftabschnitt nicht übertragen kann. Ferner wurde ein Metallrohr mit einer Faltenbalg-ähnlichen Formgebung oder mit Labyrinth-förmigen Aussparungen vorgeschlagen. Beide Ausführungsformen haben sich aber als nicht flexibel genug erwiesen, um ein weitestgehend widerstandsreduziertes Abwinkeln zu erreichen. Das Dokument US2017/333052A1 offenbart einen beispielhaften Schaft eines medizinischen Handinstruments.

### Zusammenfassung der Offenbarung

Angesichts dieser Probleme ist es die Aufgabe der vorliegenden Offenbarung, einen nach dem vorstehenden Funktionsprinzip abwinkelbaren Instrumentenschaft für medizinische Handinstrumente bereitzustellen, der sowohl einen geringen Außendurchmesser aufweist, als auch eine Drehbewegung von einem proximalen Schaftabschnitt auf einen distalen Schaftabschnitt auch bei abgewinkeltem Schaft zuverlässig und mit sensiblem Ansprechverhalten überträgt. Ferner soll die Abwinkelbewegung möglichst leichtgängig sein/bleiben.

Diese Aufgabe wird offenbarungsgemäß durch einen Schaft für ein medizinisches Handinstrument mit den Merkmalen des Anspruchs 1 gelöst. Die Aufgabe wird ferner durch ein medizinisches Handinstrument mit den Merkmalen des Anspruchs 15 gelöst.

Die vorliegende Offenbarung betrifft folglich einen Schaft eines oder für ein medizinisches Handinstrument, vorzugsweise ein chirurgisches Instrument der minimal-invasiven Bauweise, mit einem proximalen Schaftabschnitt und einem distalen Schaftabschnitt. Der distale Schaftabschnitt verdreht sich relativ zu dem proximalen Schaftabschnitt und wird dabei infolge zur Schaftlängsrichtung vorzugsweise identisch zueinander schräggestellter Stirn-Anlageseiten abgewinkelt und wieder gerade ausgerichtet. Innerhalb des Schafts verläuft u.a. ein Getriebezug mit einem Drehmoment-Übertragungsbauteil/-element im Abwinkel-Kontaktbereich des distalen und proximalen Schaftabschnitts, das eine Drehbewegung bzw. eine Rotation von dem Getriebezug im proximalen Schaftabschnitt auf den distalen Schaftabschnitt überträgt. Der Getriebezug weist folglich ein rohrförmiges Übertragungsbauteil/-element auf, das die Drehbewegung auf den distalen Schaftabschnitt überträgt und eine Abwinklung zwischen den beiden Schaftabschnitten erlaubt. Offenbarungsgemäß weist das rohrförmige Übertragungsbauteil/-element eine Anzahl an schlitzförmig ovalen Aussparungen mit jeweils spitzen Enden auf, die sich in einer Umfangsrichtung des Übertragungsbauteils/-elements erstrecken. Die Aussparungen sind ferner auf mehreren, in Bauteillängsrichtung beabstandeten Ebenen angeordnet und auf jeder Ebene in Umfangsrichtung derart beabstandet, dass die Aussparungen zweier benachbarter Ebenen in Umfangsrichtung zueinander versetzt sind.

In anderen Worten ausgedrückt besteht das Übertragungsbauteil/-element aus einem Rohrstück mit zwei axial beabstandeten Anschlussabschnitten/Endabschnitten und einem mittleren Biegeabschnitt, der eine Mehrzahl an in Umfangsrichtung sowie in Rohrlängsrichtung beabstandete, im wesentlichen ovale/elliptische Umfangsschlitze mit spitz zulaufenden Schlitzenden hat, die derart zueinander angeordnet sind, dass sich im Fall einer Abwicklung des mittleren Biegeabschnitts in die Ebene ein Diagonal-Schlitzmuster ergibt.

Der distale Schaftabschnitt ist gegenüber dem proximalen Schaftabschnitt um eine Längsachse des Schaftes verdrehbar. Die einander zugewandten Stirnseiten des distalen des proximalen Schaftabschnitts weisen jeweils einen Anstellwinkel relativ zur Schaftlängsachse auf. Wenn der distale Schaftabschnitt gegen den proximalen Schaftabschnitt verdreht wird, winkelt sich der distale Schaftabschnitt relativ zu dem proximalen Schaftabschnitt durch die beiden (gleichen) Anstellwinkel ab. Die Drehbewegung/Rotation, die für das Verdrehen des distalen Schaftabschnitts erforderlich ist, wird durch den Getriebezug in dem proximalen Schaftabschnitt initiiert, vorzugsweise durch ein Stellrad an dem proximalen Schaftabschnitt. Die Rotation wird von dem Getriebezug in dem proximalen Schaftabschnitt und dem Übertragungselement/-bauteil auf den distalen Schaftabschnitt übertragen. Das Übertragungselement/-bauteil ist im Wesentlichen ein (flexibles) Metallrohr bzw. eine Metallhülse mit einer rohrförmigen Außenwand. In der Rohrwand des Metallrohrs/Metallhülse sind die mehreren länglichen und ovalen Aussparungen/ Einschnitte/ Ausschnitte/ Schlitze eingebracht. Die Enden der ovalen/elliptischen, länglichen Aussparungen laufen jeweils spitz zusammen. Die Aussparungen verlaufen in Umfangsrichtung der Rohrwand und sind sowohl in Umfangsrichtung als auch in Längsrichtung nebeneinander angeordnet und voneinander beabstandet. Die Aussparungen sind in der Umfangsrichtung derart zueinander versetzt angeordnet, dass sich die in Längsrichtung beabstandeten Aussparungen in der Längsrichtung der länglichen Aussparungen d.h. in Umfangsrichtung gegenseitig (ausschließlich) abschnittsweise (nicht vollständig) überschneiden/überlappen. Die sich überlappenden Aussparungen bilden somit jeweils Überlappungsabschnitte. Die Überlappungsabschnitte sind kleiner/kürzer als die Erstreckung der Aussparungen in ihrer Längsrichtung bzw. der Umfangsrichtung der Rohrwand.

Der offenbarungsgemäße Schaft hat die folgenden Vorteile:
Durch die Aussparungen wird ein flexibles Übertragungselement realisiert. Das Übertragungselement hat sowohl die geforderte Torsionssteifigkeit infolge immer noch hoher zusammenhängender Anteile an Vollmaterial in der Rohrwand als auch die benötigte Flexibilität insbesondere infolge der spitz zulaufenden Oval-/Ellipsenform der Umfangsschlitze. Durch die Steifigkeit der beanspruchten Form können dünnwandigere Metall- oder Stahlrohre verwendet werden, was den Außendurchmesser des Übertragungselements verringert. Das bringt Vorteile hinsichtlich des Bauraums in dem Schaft zur Unterbringung beispielsweise des Antriebszugs für den Effektor. Durch die Ausgestaltung der Aussparungen weist das Übertragungselement also eine hohe Torsionssteifigkeit bei einer gleichzeitig guten Biegbarkeit des Übertragungselements/- bauteils auf. Da das Übertragungselement/-bauteil keine direkte Materialunterbrechung aufweist, hat es eine hohe Steifigkeit.

Durch die ovalen/elliptischen Aussparungen ist eine Stauchung des Übertragungselements in sämtlichen Winkelungen möglich. Das Übertragungselement ist dadurch in alle Richtungen flexibel. Durch die überlappenden Aussparungen bleibt kein (durchgehender) Mittelsteg bestehen. Dadurch bekommt das Übertragungselement eine federnde Wirkung. Die ovalen Aussparungen verbessern ferner das Bruchverhalten des Übertragungselements. Die Kerbspannung ist bei den ovalen Aussparungen geringer als bei einer scharfen Kante bei einer geraden Aussparung.

Vorteilhafte Weiterbildungen der Offenbarung sind Gegenstand der beigefügten Unteransprüche.

Nach einem optionalen Aspekt der Offenbarung ist die rohrförmige Außenwand des Übertragungselements derart ausgeschnitten, dass das Übertragungselement in Längsrichtung biegbar ist. Gleichzeitig ist das Übertragungselement torsionssteif. Durch das Abwinkeln des distalen Schaftabschnitts wird das Übertragungselement in seiner Längsrichtung gebogen. Daher ist es vorteilhaft, wenn das Übertragungselement in seiner Längsachse biegbar ist. Das Übertragungselement überträgt zusätzlich die Rotation von dem Getriebezug auf den distalen Schaftabschnitt. Das Übertragungselement bewirkt somit eine Drehmomentübertragung. Daher ist eine hohe Torsionssteifigkeit des Übertragungselements von Vorteil.

Nach einem weiteren optionalen Aspekt der Offenbarung überlappen sich die Anzahl an Aussparungen in den Überlappungsabschnitten und eine Länge der Überlappungsabschnitte beträgt vorzugsweise jeweils ein Drittel der Länge der Aussparungen. Durch diese Überlappung wird die hohe Biegbarkeit bei gleichzeitig hoher Torsionssteifigkeit sichergestellt.

Nach einem weiteren optionalen Aspekt der Offenbarung sind die Überlappungsabschnitte in einem Endbereich der Aussparungen positioniert, der den Bereich der größten Dicke der Aussparungen ausschließt. Dadurch können die mehreren Aussparungen näher beieinander angeordnet werden, was die Biegbarkeit bzw. Flexibilität des Übertragungselements verbessert/erhöht.

Vorzugsweise ist das Übertragungsbauteil/-element an dem proximalen Endabschnitt rotationsfest mit einer Buchse verbunden, die mit einem Ritzel verbunden ist, das von einem Hohlrad des Getriebezugs angetrieben wird. Die Buchse treibt das Übertragungsbauteil/-element an und überträgt die Drehbewegung des Getriebezugs auf das Übertragungsbauteil/-element. Dadurch kann das Drehmoment von dem Getriebezug im proximalen Schaftabschnitt über das Hohlrad, das Ritzel und die Buchse auf das Übertragungsbauteil/-element übertragen werden. Das Übertragungsbauteil/-element wiederum kann das Drehmoment vom proximalen Schaftabschnitt zu dem distalen Schaftabschnitt übertragen. Somit kann eine einfache und fehlertolerante Drehmomentübertragung bereitgestellt werden.

Nach einem weiteren optionalen Aspekt der Offenbarung ist das Übertragungselement an einem proximalen Ende/Endabschnitt rotationsfest mit der Buchse verbunden, die die Drehbewegung/Rotation des Getriebezugs auf das Übertragungselement überträgt. Das rohrförmige Übertragungselement hat ein distales Ende/Endabschnitt und das proximale Ende/Endabschnitt. Über das Übertragungselement wird die Rotation dann weiter auf den distalen Schaftabschnitt übertragen.

Nach einem weiteren optionalen Aspekt der Offenbarung weist das Übertragungselement an dem proximalen Ende mehrere Langlöcher bzw. zur proximalen Stirnseite hin offene Längsschlitze auf, die bevorzugt diametral gegenüberliegen und die dafür vorgesehen und ausgebildet sind, jeweils eine Nase bzw. Eingriffsnase der Buchse aufzunehmen, die von der Umfangswand der Buchse radial nach innen vorstehen. Die Längsschlitze sind derart ausgestaltet und vorbereitet, dass die vorstehenden Eingriffsnasen in die Langlöcher eingreifen und sind an dem proximalen Ende offen. Dadurch sind die Eingriffsnasen in den Langlöchern axial beweglich gelagert und gleichzeitig in Umfangsrichtung fest/ radial unbeweglich bzw. rotationsfest. Die Rotation des Gewindezugs kann so auf das Übertragungselement übertragen werden und die Verbindung ist statisch bestimmt.

Nach einem weiteren optionalen Aspekt der Offenbarung weist der Getriebezug das Hohlrad auf, das mit dem bezüglich der Mittelachse des proximalen Schaftabschnitts dezentral positionierten Ritzel in Kämmeingriff steht, das wiederum mit der axial sich anordnenden Buchse fest verbunden ist. Das Hohlrad ist mit dem Stellrad wirkverbunden und kann somit über das Stellrad angetrieben werden. Somit wird eine durch das Stellrad initiierte Rotation des Getriebezugs von dem Hohlrad über das Ritzel auf die Buchse übertragen und von dieser auf das rotationsfest mit der Buchse verbundene Übertragungselement/-bauteil weitergegeben.

Nach einem weiteren optionalen Aspekt der Offenbarung ist die Buchse durch eine Presspassung mit dem Ritzel verbunden. Dadurch wird sichergestellt, dass die Buchse und das Ritzel rotationsfest und bevorzugt axialfest verbunden sind.

Nach einem weiteren optionalen Aspekt der Offenbarung weist der Getriebezug eine exzentrische Sicherungsbuchse auf, die innerhalb eines Außenrohres des proximalen Schaftabschnitts angeordnet ist und in der die Buchse drehbar gelagert ist. Die exzentrische Sicherungsbuchse hat folglich ein axial sich erstreckendes, dezentral ausgebildetes Lagerloch, in welchem die Buchse gleitgelagert ist. Durch die exzentrische Sicherungsbuchse ist die Buchse und damit das Übertragungselement nicht genau in der Mitte des Außenrohrs des proximalen Schaftabschnitts angeordnet. Durch diese dezentrale Anordnung der Buchse und des Übertragungselements wird der Antriebszug für den distalen Effektor, der in dem rohrförmigen Übertragungselement geführt wird, über eine längere Strecke gebogen und hat damit eine geringe Belastung durch Biegung.

Nach einem weiteren optionalen Aspekt der Offenbarung ist das Übertragungselement/-bauteil an einem distalen Ende fest mit einer Verstellbuchse verbunden, die die Drehbewegung des Übertragungselements auf den distalen Schaftabschnitt überträgt, der eine Schaftspitze hat oder eine Schaftspitze darstellt. Durch die feste Verbindung des Übertragungselements mit der Verstellbuchse wird die Rotation des Getriebezugs im proximalen Schaftabschnitt durch das Übertragungselement auf den distalen Schaftabschnitt übertragen. Die Verstellbuchse weist vorzugsweise einen axial sich erstreckenden, dezentral platzierten Stift auf, der formschlüssig in den distalen Schaftabschnitt eingreift, sich mit der Verstellbuchse dreht und damit den distalen Schaftabschnitt mit dreht.

Nach einem weiteren optionalen Aspekt der Offenbarung ist das Übertragungselement mit einer Innenseite der Verstellbuchse verschweißt. Durch Schweißen wird eine rotationsfeste Verbindung realisiert.

Nach einem weiteren optionalen Aspekt der Offenbarung ist das Übertragungselement um 22,5° bezüglich des proximalen Schaftabschnitts abgewinkelt, wenn dieser in gerader Konstruktionslage zum distalen Schaftabschnitt ausgerichtet ist. Der distale Schaftabschnitt kann nunmehr im Vergleich zu dem proximalen Schaftabschnitt um 45° abgewinkelt werden, wenn dieser um das Übertragungselement als Rotationsachse dreht. Dies wird durch die Biegung des Übertragungselements um 22,5° ermöglicht.

Nach einem weiteren optionalen Aspekt der Offenbarung hat der Schaft einen Außendurchmesser von weniger als 5,6 mm. Durch die konstruktive Ausgestaltung des Übertragungselements wird der geringe Außendurchmesser des Schafts ermöglicht. Durch die Ausgestaltung der Aussparungen ist ein biegbares Übertragungselement mit der notwendigen Torsionssteifigkeit und einem geringen Durchmesser ermöglicht.

Nach einem weiteren optionalen Aspekt der Offenbarung ist das Übertragungselement einteilig. Die Aussparungen werden beispielsweise in Diagonalmuster aus einem ebenen Blech ausgestanzt, das anschließend zu dem rohrförmigen Übertragungselement geformt und ggf. geschweißt wird. Durch die einteilige Ausgestaltung sind keine Gelenke, Verbindungsglieder oder Ähnliches zwischen einzelnen Elementen des Übertragungselements notwendig. Dadurch können Montagekosten eingespart werden.

In anderen Worten ausgedrückt kann ein Schaft eines oder für ein medizinisches Handinstrument, vorzugsweise ein chirurgisches Instrument der minimal-invasiven Bauweise vorgesehen sein, mit einem proximalen Schaftabschnitt und einem distalen Schaftabschnitt, der sich relativ zu dem proximalen Schaftabschnitt verdreht und dabei abwinkelt, und einem Getriebezug, der innerhalb des Schafts angeordnet ist und eine Drehbewegung des Getriebezugs im proximalen Schaftabschnitt auf den distalen Schaftabschnitt überträgt, wobei der Getriebezug ein rohrförmiges Übertragungsbauteil/-element aufweist, das die Drehbewegung auf den distalen Schaftabschnitt überträgt und eine Abwinklung zwischen beiden Schaftabschnitten erlaubt, wobei das Übertragungsbauteil/-element ein Rohrstück mit zwei axial beabstandeten Endabschnitten und einem mittleren Biegeabschnitt hat oder ist und wobei das Übertragungsbauteil/-element an dem proximalen Endabschnitt (26) rotationsfest mit einer Buchse verbunden ist, die mit einem Ritzel verbunden ist, das von einem Hohlrad des Getriebezugs angetrieben wird, wobei die Buchse das Übertragungsbauteil/-element antreibt die Drehbewegung des Getriebezugs auf das Übertragungsbauteil/-element überträgt und in dem mittleren Biegeabschnitt eine Mehrzahl an in Umfangsrichtung sowie in Rohrlängsrichtung beabstandeten, im wesentlichen ovalen/elliptischen sowie in Umfangsrichtung sich erstreckenden Aussparungen mit spitz zulaufenden Aussparungsenden ausgeformt sind, die derart zueinander angeordnet sind, dass sich im Fall einer Abwicklung des mittleren Biegeabschnitts in die Ebene ein Diagonal-Aussparungsmuster ergibt.

Für den Schaft kann es vorgesehen sein, dass das Übertragungsbauteil/-element an dem proximalen Endabschnitt mehrere Längsschlitze aufweist, die sich an dem proximalen Endabschnitt öffnen und dafür vorgesehen und ausgebildet sind, jeweils eine radial von der Buchse vorstehende Eingriffsnase aufzunehmen.

Für den Schaft kann es vorgesehen sein, dass der Getriebezug die Buchse, ein Hohlrad und das Ritzel aufweist, wobei das Ritzel von dem Hohlrad angetrieben wird.

Für den Schaft kann es vorgesehen sein, dass die Buchse durch eine Presspassung mit dem Ritzel verbunden ist.

Für den Schaft kann es vorgesehen sein, dass sich die axialbeabstandeten Aussparungen in Umfangsrichtung des Übertragungsbauteil/-elements in Überlappungsabschnitten überlappen und eine Länge der Überlappungsabschnitte vorzugsweise jeweils ein Drittel der Länge der Aussparungen beträgt.

Für den Schaft kann es vorgesehen sein, dass die Überlappungsabschnitte einen Bereich der größten Dicke der Aussparungen ausgrenzen.

Für den Schaft kann es vorgesehen sein, dass der proximale Schaftabschnitt eine exzentrische Sicherungsbuchse aufweist, die innerhalb eines Außenrohres des proximalen Schaftabschnitts fest angeordnet ist und in der die Buchse drehbar sowie dezentral bezüglich einer Mittelachse des proximalen Schaftabschnitts gelagert ist.

Für den Schaft kann es vorgesehen sein, dass das Übertragungsbauteil/-element an einem distalen Ende fest mit einer Verstellbuchse verbunden ist, die die Drehbewegung des Übertragungsbauteil/-elements auf den distalen Schaftabschnitt überträgt.

Für den Schaft kann es vorgesehen sein, dass das Übertragungsbauteil/-element mit einer Innenseite der Verstellbuchse verschweißt ist.

Für den Schaft kann es vorgesehen sein, dass das Übertragungsbauteil/-element in gerader Konstruktionslage der beiden Schaftabschnitte in einem Winkel von 22,5° abgewinkelt verbaut ist, derart, dass der proximale Endabschnitt des Übertragungsbauteil/-elements parallel zur Mittelachse des proximalen Schaftabschnitts und der distale Endabschnitt des Übertragungsbauteil/-elements in dem Winkel von 22,5° bezüglich der Mittelachse des distalen Schaftabschnitts ausgerichtet ist.

Für den Schaft kann es vorgesehen sein, einen Außendurchmesser von weniger als 5,6 mm anzuordnen.

Für den Schaft kann es vorgesehen sein, dass das Übertragungsbauteil/-element einteilig ist.

Die Aufgabe der Offenbarung wird ferner durch ein medizinisches Handinstrument mit einem Schaft nach einem der vorstehenden Aspekte und einem proximalen Handstück gelöst.

### Kurzbeschreibung der Figuren

Fig. 1 zeigt einen Längsschnitt durch einen offenbarungsgemäßen Schaft eines medizinischen Handstücks;
Fig. 2 zeigt eine isometrische Darstellung eines Übertragungselements gemäß einer ersten Ausführungsform der Offenbarung;
Fig. 3 zeigt das Übertragungselement gemäß der ersten Ausführungsform mit einer Verstellbuchse;
Fig. 4 zeigt einen Querschnitt durch den offenbarungsgemäßen Schaft;
Fig. 5 zeigt eine isometrische Darstellung einer Buchse, die mit einem Ritzel verbunden ist;
Fig. 6 zeigt einen Längsschnitt durch einen offenbarungsgemäßen geraden Schaft;
Fig. 7 zeigt einen Längsschnitt durch einen offenbarungsgemäßen abgewinkelten Schaft;
Fig. 8 zeigt eine Draufsicht auf ein ausgerolltes Übertragungselement mit einer Anzahl an Aussparungen gemäß der ersten Ausführungsform der Offenbarung;
Fig. 9 zeigt eine Seitenansicht eines Übertragungselements gemäß einer zweiten Ausführungsform der Offenbarung;
Fig. 10 zeigt eine Seitenansicht eines Übertragungselements gemäß einer dritten Ausführungsform der Offenbarung;
Fig. 11 zeigt eine Seitenansicht eines Übertragungselements gemäß einer vierten Ausführungsform der Offenbarung;
Fig. 12 zeigt eine Seitenansicht eines Übertragungselements gemäß einer fünften Ausführungsform der Offenbarung; und
Fig. 13 zeigt ein offenbarungsgemäßes Handinstrument.

### Beschreibung der Ausführungsformen

Nachstehend werden Ausführungsformen der vorliegenden Offenbarung auf der Basis der zugehörigen Figuren beschrieben.

### Erste Ausführungsform

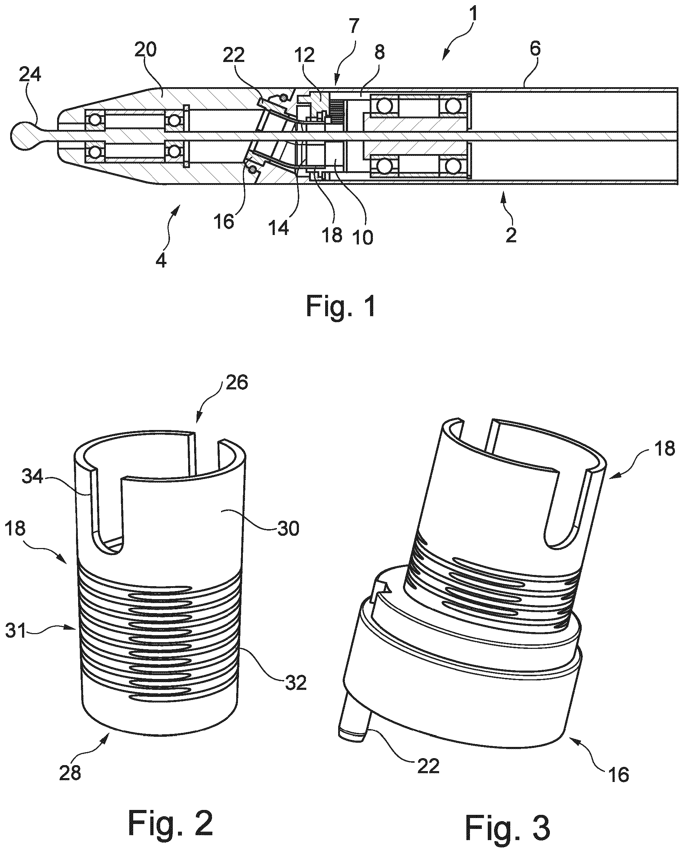

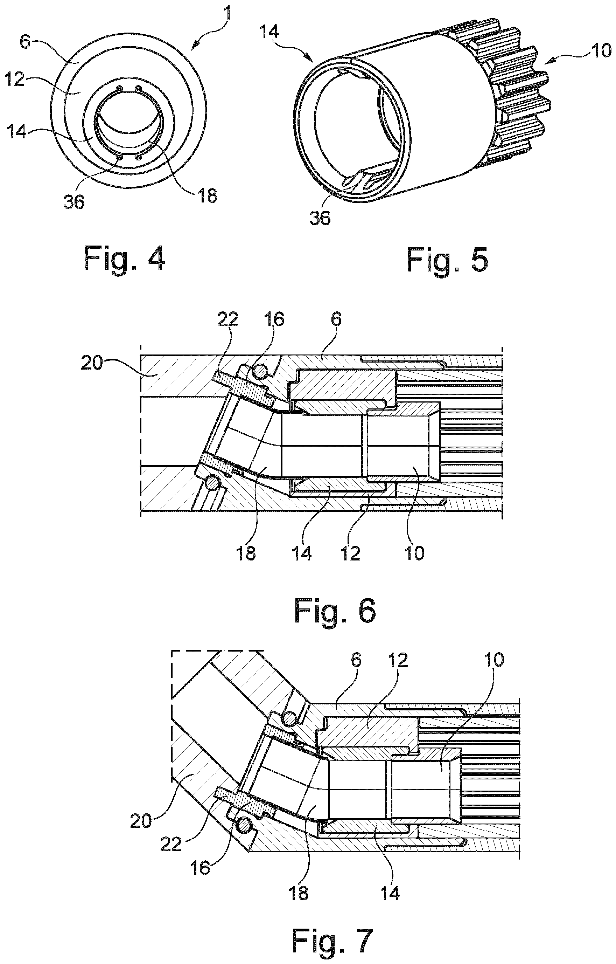

Fig. 1 zeigt einen Längsschnitt durch einen offenbarungsgemäßen (Instrumenten-) Schaft 1. Der Schaft 1 weist einen proximalen Schaftabschnitt 2 und einen distalen Schaftabschnitt 4 auf. Der proximale Schaftabschnitt 2 hat ein Außenrohr 6. In dem Schaft 1 und innerhalb des Außenrohrs 6 ist ein Getriebezug 7 angeordnet. Der Getriebezug 7 weist ein Hohlrad 8, ein Ritzel 10, eine exzentrische Sicherungsbuchse 12 und eine Buchse 14 auf. Das Hohlrad 8 ist innerhalb des Außenrohrs 6 angeordnet und wird von einem ggf. manuell betätigbaren Stellrad (nicht dargestellt) angetrieben. Eine Innenverzahnung des Hohlrads 8 kämmt mit einer Außenverzahnung des Ritzels 10. Dadurch treibt das Hohlrad 8 das Ritzel 10 an. Das Ritzel 10 ist fest mit der Buchse 14 axial verbunden (wie in Fig. 5 gezeigt), die innerhalb der exzentrischen Sicherungsbuchse 12 angeordnet ist und damit ebenfalls dezentral bezüglich der Mittelachse des proximalen Schaftabschnitts positioniert wird. Der Getriebezug 7 weist ferner eine Verstellbuchse 16 und ein Übertragungsbauteil/-element 18 auf.

Der distale Schaftabschnitt 4 weist eine distale Schaftspitze 20 auf. Die Verstellbuchse 16 weist einen Stift 22 auf, der dezentral zur Mittelachse der Verstellbuchse 16 angeordnet ist und sich axial in Richtung distal von dieser erstreckt. Der Stift 22 greift formschlüssig in ein proximales Ende des distalen Schaftabschnitts 4 ein. Dadurch sind die Verstellbuchse 16 und die distale Schaftspitze 20 über den distalen Schaftabschnitt 4 rotationsfest miteinander verbunden. Das Übertragungselement 18 verbindet axial die Verstellbuchse 16 und die Buchse 14. Damit überträgt der Getriebezug 7 und besonders das Übertragungselement 18 eine Rotation ausgehend vom (nicht dargestellten) Stellrad auf den distalen Schaftabschnitt 4. Innerhalb des proximalen Schaftabschnitts 2 und des distalen Schaftabschnitts 4 verläuft ein Antriebsstrang für einen an der Schaftspitze 20 gelagerten Effektor 24, z.B. Fräser oder Bohrer, wobei der Antriebsstrang oder Antriebszug abschnittsweise durch den Getriebezug 7 insbesondere Übertragungselement 18, Ritzel 10 und Hohlrad 8 zentral hindurchgeführt ist.

Fig. 2 zeigt das Übertragungselement 18 gemäß einer ersten Ausführungsform. Das Übertragungselement 18 ist im Wesentlichen ein axial abschnittsweise flexibles Rohr und weist ein proximales Ende/Endabschnitt bzw. Anschlussabschnitt 26 und ein distales Ende/Endabschnitt bzw. einen Anschlussabschnitt 28 auf. In einer Rohrwand 30 des rohrförmigen Übertragungselements 18 weist dieses in einem axialen mittleren Biegeabschnitt 31 eine Anzahl/Mehrzahl an ovalen, länglichen Aussparungen 32 auf. Die Aussparungen 32 haben annähernd die Form von ovalen Spalten/Schlitzen und Aussparungsenden laufen spitz zusammen. Die Aussparungen 32 erstrecken sich in deren Längsrichtung in Umfangsrichtung des rohrförmigen Übertragungselements 18 und sind von den jeweiligen Endabschnitten 26, 28 des Übertragungselements 18 in Längsrichtung beabstandet und in Richtung des proximalen Endes/Endabschnitts 26 versetzt. Die Aussparungen 32 sind in Längsrichtung des Übertragungselements 18 bzw. axial voneinander beabstandet und sind in Umfangsrichtung des Übertragungselements zueinander versetzt, derart, dass sich in einer Abwinklung der Rohrwand in einer Ebene ein Diagonalmuster der Aussparungen 32 ergibt. Die Ausgestaltung der Aussparungen 32 wird nachfolgend detailliert erläutert.

An dem proximalen Ende 26 weist das Übertragungselement 18 zwei sich gegenüberliegende Langlöcher/Längsschlitze 34 auf. Die Längsschlitze 34 verlaufen in Längsrichtung des Übertragungselements 18. D.h. die Längsschlitze 34 erstrecken sich in Richtung des mittleren Biegebereichs 31 mit den Aussparungen 32, ist von dem mittleren Biegebereich 31 dabei aber beabstandet.

Fig. 3 zeigt das Übertragungselement 18, das an seinem distalen Ende 28 mit der Verstellbuchse 16 verbunden ist. Das Übertragungselement 18 ist vorzugsweise mit der Verstellbuchse 16 verschweißt. Die Verstellbuchse 16 ist im Wesentlichen ein metallener Ring. Die Innenseite des Rings hat in etwa den Durchmesser des Übertragungselements 18 und das Übertragungselement 18 ist mit der Innenseite des Rings verbunden. An ihrer distalen Seite weist die Verstellbuchse 16 den Stift 22 auf.

Der Stift 22 greift formschlüssig in den distalen Schaftabschnitt 4 ein. Wenn sich das Übertragungselement 18 dreht, dreht sich auch die Verstellbuchse 16 mit. Der Stift 22 dreht wiederum über den distalen Schaftabschnitt 4 die distale Schaftspitze 20 mit. So überträgt das Übertragungselement 18 die Rotation des Getriebezugs 7 im proximalen Schaftabschnitt 2 auf den distalen Schaftabschnitt 4.

Fig. 4 zeigt einen Querschnitt durch den Schaft 1. Das Übertragungselement 18 ist dabei rotationsfest in der Buchse 14 angeordnet. Dabei ist das Übertragungselement 18 axial beweglich. In einer radialen Richtung greifen radial vorstehende Eingriffsnasen 36 der Buchse 14 in die Längsschlitze 34 des Übertragungselements 18 ein. Durch die vorstehenden Eingriffsnasen 36 wird die Rotation der Buchse 14 auf das Übertragungselement 18 übertragen. Die Buchse 14 ist fest mit dem Ritzel 10 verbunden, wie in Fig. 5 gezeigt. Die Eingriffsnasen 36 liegen einander gegenüber und stehen aus einer Umfangswand der Buchse 14 radial nach innen vor. Dabei ist die Buchse 14 mit dem Ritzel 10 vorzugsweise durch einen Presssitz verbunden. Die Buchse 14 ist in der exzentrischen Sicherungsbuchse 12, die eine Distanzhülse bzw. ein Abstandhalter ist, in dem Außenrohr 6 des proximalen Schaftabschnitts 2 gelagert.

Fig. 6 zeigt einen Längsschnitt durch den Schaft 1. Der Schaft 1 ist in dieser Ansicht gerade. Es ist gezeigt, wie das Übertragungselement 18 an dem proximalen Ende/Endabschnitt 26 mit der Buchse 14 und an dem distalen Ende/Endabschnitt 28 fest mit der Innenseite der Verstellbuchse 16 verbunden ist. Dadurch ist eine Drehmomentübertragung von dem Ritzel 10 über die Buchse 14 auf das Übertragungselement 18 und weiter auf die Verstellbuchse 16 und den distalen Schaftabschnitt 4 möglich. Fig. 7 zeigt einen Längsschnitt durch einen abgewinkelten Schaft 1. Es ist gezeigt, dass die Verstellbuchse 16 mit dem Stift 22 im Vergleich zur Fig. 6 um 180° um deren Längsachse gedreht ist. Dadurch ist auch die distale Schaftspitze 20 um 180° verdreht. Durch angestellte Abschlussflächen des distalen Schaftabschnitts 4 und des Außenrohrs 6 des proximalen Schaftabschnitts 2 wird der distale Schaftabschnitt 4 relativ zu dem proximalen Schaftabschnitt 2 bei entsprechender Drehung um dessen Längsachse gleichzeitig abgewinkelt. Der distale Schaftabschnitt 4 und der proximale Schaftabschnitt 2 weisen in dieser Stellung einen Winkel von 45° zueinander auf.

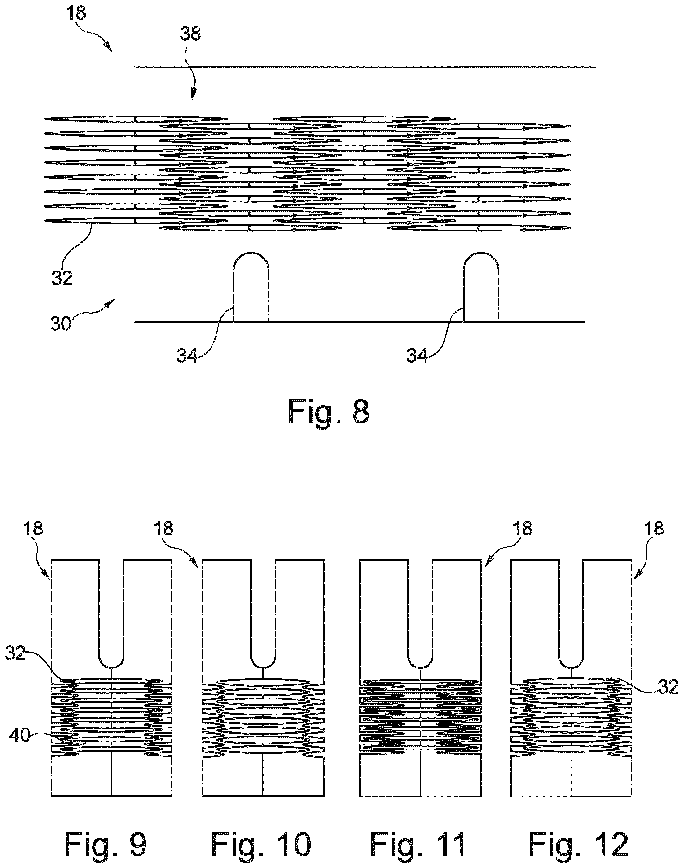

Fig. 8 zeigt das Blech/ Außenrohr/ Mantelmaterial 30 des Übertragungselements 18 in einer Abwicklung in einer Ebene. Das Übertragungselement 18 weist die Anzahl an Aussparungen 32 auf. Die Aussparungen 32 sind oval geformt und verlaufen quer zur Längsrichtung des Übertragungselements 18 bzw. in der Umfangsrichtung des rohrförmigen Übertragungselements 18. Das Übertragungselement 18 weist Überlappungsabschnitte 38 auf, in denen sich die Aussparungen 32 in ihrer Längsrichtung bzw. in der Umfangsrichtung des rohrförmigen Übertragungselements 18 gegenseitig überlappen. Dabei ist die Länge der Überlappungsabschnitte 38 geringer als die größere Erstreckung der Aussparungen 32. Die Länge der Überlappungsabschnitte 38 beträgt vorzugsweise ein Drittel der Länge der Aussparungen.

Im Nachfolgenden werden Varianten bezüglich der Formgebung der Aussparungen 32 unter Verweis auf die Figuren 9 bis 12 beschrieben.

### Zweite Ausführungsform

Fig. 9 zeigt eine Seitenansicht eines Übertragungselements 18 gemäß einer zweiten Ausführungsform. Wie hieraus entnehmbar ist, kann die Dicke der Aussparungen in Längsrichtung des Verbindungselements so gewählt werden, dass die Materialstege zwischen den axialbeabstandeten Aussparungen dicker (oder auch dünner) werden. Das bedeutet, dass quasi durch die Wahl der Aussparungs-/Schlitzbreite bei gleicher Schlitzanzahl und axialem Abstand der einzelnen Schlitze die Biegesteifigkeit eingestellt werden kann. In Fig. 9 sind die Schlitzbreiten klein und damit die Stegbreite zwischen den jeweils axial benachbarten Schlitzen groß.

### Dritte Ausführungsform

Im Gegensatz zu Fig. 9 zeigt Fig. 10 eine Seitenansicht eines Übertragungselements 18 gemäß einer dritten Ausführungsform, bei der die Breite der ovalen Aussparung 32 in axialer Richtung des Übertragungselements gegenüber der zweiten Ausführungsform erhöht ist (wodurch demzufolge die Mittelstege schmäler werden). Dadurch wird eine Kerbspannung an den Seiten der Aussparungen 32 reduziert. Ferner wurde die Länge der Aussparungen 32 in Umfangsrichtung reduziert, um eine ausreichende Wandstärke zwischen den in Umfangsrichtung beabstandeten ovalen Aussparungen 32 zu gewährleisten.

### Vierte Ausführungsform

Fig. 11 zeigt eine Seitenansicht eines Übertragungselements 18 gemäß einer vierten Ausführungsform. Für die vierte Ausführungsform ist die Länge der ovalen Aussparungen 32 in Umfangsrichtung erhöht. Dadurch wird eine größere Überschneidung der Aussparungen 32 ermöglicht und das Übertragungselement 18 gewinnt an Flexibilität. Die Dicke der Aussparungen 32 ist im Gegensatz dazu reduziert. Dadurch wird eine ausreichende Wandstärke zwischen den einzelnen Aussparungen 32 gewährleistet. Die Kerbspannung wird dadurch erhöht. Damit stellt die vierte Ausführungsform das genaue Gegenteil der dritten Ausführungsform dar.

### Fünfte Ausführungsform

Fig. 12 zeigt eine Seitenansicht eines Übertragungselements 18 gemäß einer fünften Ausführungsform. Dabei weisen alle Mittelstege 40 zwischen den Aussparungen 32 die gleichen Wandstärken auf. Dadurch erhöht sich die Flexibilität des Übertragungselements 18, ohne dass sich die Kerbspannung erhöht.

Die Wand des Übertragungselements 18 ist vorzugsweise 0,2 mm dick. Ein Außendurchmesser des Übertragungselements 18 beträgt vorzugsweise 2,5 mm. Vorzugsweise besteht das Übertragungselement 18 aus Stahl 1.4301. Weiterhin vorzugsweise ist das Übertragungselement 18 aus einer Formgedächtnislegierung, wie beispielsweise Nitinol. Das fördert die Flexibilität und die Formstabilität. Weiterhin ist denkbar, das Übertragungselement 18 aus einer Hochleistungsstahllegierung wie beispielsweise 1.4197 zu fertigen, was das übertragbare Drehmoment erhöht.

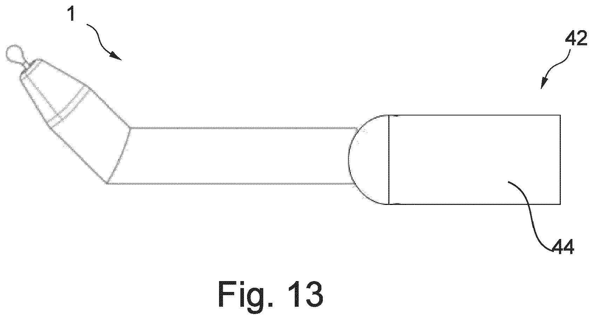

Fig. 13 zeigt ein offenbarungsgemäßes Handinstrument 42 mit einem Schaft 1 und einem Handstück 44. Das Handinstrument 42 ist beispielsweise ein Bohrer oder ein Fräser.

### Bezugszeichenliste

- 1: Schaft
- 2: proximaler Schaftabschnitt
- 4: distaler Schaftabschnitt
- 6: Außenrohr
- 7: Getriebezug
- 8: Hohlrad
- 10: Ritzel
- 12: exzentrische Sicherungsbuchse
- 14: Buchse
- 16: Verstellbuchse
- 18: Übertragungselement
- 20: distale Schaftspitze
- 22: Stift
- 24: Fräser
- 26: proximales Ende des Übertragungselements
- 28: distales Ende des Übertragungselements
- 30: Außenwand des Übertragungselements
- 31: mittlerer Biegeabschnitt
- 32: Aussparung
- 34: Längsschlitz
- 36: Eingriffsnase
- 38: Überlappungsabschnitte
- 40: Mittelsteg
- 42: Handinstrument
- 44: Handstück

## Patentansprüche

1. Schaft (1) eines oder für ein medizinisches Handinstrument (42), vorzugsweise ein chirurgisches Instrument der minimal-invasiven Bauweise, mit einem proximalen Schaftabschnitt (2) und einem distalen Schaftabschnitt (4), der sich relativ zu dem proximalen Schaftabschnitt (2) verdreht und dabei abwinkelt, und
einem Getriebezug (7), der innerhalb des Schafts (1) angeordnet ist und eine Drehbewegung des Getriebezugs (7) im proximalen Schaftabschnitt (2) auf den distalen Schaftabschnitt (4) überträgt,
wobei der Getriebezug (7) ein rohrförmiges Übertragungsbauteil/-element (18) aufweist, das die Drehbewegung auf den distalen Schaftabschnitt (4) überträgt und eine Abwinklung zwischen beiden Schaftabschnitten (2, 4) erlaubt,
wobei
das Übertragungsbauteil/-element (18) ein Rohrstück mit zwei axial beabstandeten Endabschnitten (26, 28) und einem mittleren Biegeabschnitt (31) hat oder ist, in welchem eine Mehrzahl an in Umfangsrichtung sowie in Rohrlängsrichtung beabstandeten, im wesentlichen ovalen/elliptischen sowie in Umfangsrichtung sich erstreckenden Aussparungen (32) mit spitz zulaufenden Aussparungsenden ausgeformt sind, die derart zueinander angeordnet sind, dass sich im Fall einer Abwicklung des mittleren Biegeabschnitts (31) in die Ebene ein Diagonal-Aussparungsmuster ergibt.

2. Schaft (1) nach Anspruch 1, **dadurch gekennzeichnet, dass** sich die axialbeabstandeten Aussparungen (32) in Umfangsrichtung des Übertragungsbauteils/- elements (18) in Überlappungsabschnitten (38) überlappen und eine Länge der Überlappungsabschnitte (38) vorzugsweise jeweils ein Drittel der Länge der Aussparungen (32) beträgt.

3. Schaft (1) nach Anspruch 2, **dadurch gekennzeichnet, dass** die Überlappungsabschnitte (38) einen Bereich der größten Dicke der Aussparungen (32) ausgrenzen.

4. Schaft (1) nach einem der Ansprüche 1 bis 3, **dadurch gekennzeichnet, dass** das Übertragungsbauteil/-element (18) an einem proximalen Endabschnitt (26) rotationsfest mit einer Buchse (14) verbunden ist, die die Drehbewegung vom Getriebezug (7) innerhalb des proximalen Schaftabschnitts (2) auf das Übertragungselement (18) überträgt.

5. Schaft (1) nach Anspruch 4, **dadurch gekennzeichnet, dass** das Übertragungsbauteil/-element (18) an dem proximalen Endabschnitt (26) mehrere Längsschlitze (34) aufweist, die sich an dem proximalen Endabschnitt (26) öffnen und dafür vorgesehen und ausgebildet sind, jeweils eine radial von der Buchse (14) vorstehende Eingriffsnase (36) aufzunehmen.

6. Schaft (1) nach Anspruch 4 oder 5, **dadurch gekennzeichnet, dass** der Getriebezug (7) die Buchse (14), ein Hohlrad (8) und ein Ritzel (10) aufweist, das vom Hohlrad (8) angetrieben wird, und die Buchse (14) fest mit dem Ritzel (10) axial verbunden ist.

7. Schaft (1) nach Anspruch 6, **dadurch gekennzeichnet, dass** die Buchse (14) durch eine Presspassung mit dem Ritzel (10) verbunden ist.

8. Schaft (1) nach einem der Ansprüche 4 bis 7, **dadurch gekennzeichnet, dass** der proximale Schaftabschnitt (2) eine exzentrische Sicherungsbuchse (12) aufweist, die innerhalb eines Außenrohres (6) des proximalen Schaftabschnitts (2) fest angeordnet ist und in der die Buchse (14) drehbar sowie dezentral bezüglich einer Mittelachse des proximalen Schaftabschnitts (2) gelagert ist.

9. Schaft (1) nach einem der Ansprüche 1 bis 8, **dadurch gekennzeichnet, dass** das Übertragungsbauteil/-element (18) an einem distalen Ende (28) fest mit einer Verstellbuchse (16) verbunden ist, die die Drehbewegung des Übertragungsbauteils/- elements (18) auf den distalen Schaftabschnitt (4) überträgt.

10. Schaft (1) nach Anspruch 9, **dadurch gekennzeichnet, dass** das Übertragungsbauteil/-element (18) mit einer Innenseite der Verstellbuchse (16) verschweißt ist.

11. Schaft (1) nach einem der Ansprüche 1 bis 10, **dadurch gekennzeichnet, dass** das Übertragungsbauteil/-element (18) in gerader Konstruktionslage der beiden Schaftabschnitte (2, 4) in einem Winkel von 22,5° abgewinkelt verbaut ist, derart, dass der proximale Endabschnitt (26) des Übertragungsbauteils/-elements (18) parallel zur Mittelachse des proximalen Schaftabschnitts (2) und der distale Endabschnitt (28) des Übertragungsbauteils/-elements (18) in dem Winkel von 22,5° bezüglich der Mittelachse des distalen Schaftabschnitts (4) ausgerichtet ist.

12. Schaft (1) nach einem der Ansprüche 1 bis 11, **gekennzeichnet durch** einen Außendurchmesser von weniger als 5,6 mm.

13. Schaft (1) nach einem der Ansprüche 1 bis 12, **dadurch gekennzeichnet, dass** das Übertragungsbauteil/-element (18) einteilig ist.

14. Medizinisches Handinstrument (42) mit einem Schaft (1) nach einem der Ansprüche 1 bis 13 und einem proximalen Handstück (44).

## Claims

1. A shaft (1) of or for a medical hand instrument (42), preferably a surgical instrument of the minimally invasive type, having a proximal shaft portion (2) and a distal shaft portion (4) which rotates and thereby assumes an angle relative to the proximal shaft portion (2), and
a train of gears (7), which is arranged within the shaft (1) and transmits a rotary movement of the train of gears (7) in the proximal shaft portion (2) to the distal shaft portion (4),
wherein the train of gears (7) has a tubular transmission component/element (18) which transmits the rotational movement to the distal shaft portion (4) and permits angular deflection between the two shaft portions (2, 4),
wherein
the transmission component/element (18) has or is a tube piece with two axially spaced end portions (26, 28) and a central bending portion (31), in which a plurality of substantially oval/elliptical recesses (32), which are spaced apart in the circumferential direction and in the longitudinal direction of the tube and extend in the circumferential direction, are formed with tapered recess ends which are arranged relative to each other in such a way that a diagonal recess pattern results when the central bending portion (31) is unwound in the plane.

2. The shaft (1) according to claim 1, **characterized in that** the axially spaced recesses (32) overlap in overlapping portions (38) in the circumferential direction of the transmission component/element (18), and a length of the overlapping portions (38) is preferably one third of the length of the recesses (32) in each case.

3. The shaft (1) according to claim 2, **characterized in that** the overlapping portions (38) delimit a region of the greatest thickness of the recesses (32).

4. The shaft (1) according to one of claims 1 to 3, **characterized in that** the transmission component/element (18) is connected at a proximal end portion (26) in a rotationally fixed manner to a bushing (14), which transmits the rotary movement from the train of gears (7) within the proximal shaft portion (2) to the transmission element (18).

5. The shaft (1) according to claim 4, **characterized in that** the transmission component/element (18) has a plurality of longitudinal slits (34) at the proximal end portion (26), wherein the slits open at the proximal end portion (26) and are intended and configured to each receive an engagement nose (36) projecting radially from the bushing (14).

6. The shaft (1) according to claim 4 or 5, **characterized in that** the train of gears (7) has the bushing (14), a hollow wheel (8) and a pinion (10) which is driven by the hollow wheel (8), and the bushing (14) is fixedly connected axially to the pinion (10).

7. The shaft (1) according to claim 6, **characterized in that** the bushing (14) is connected to the pinion (10) by an interference fit.

8. The shaft (1) according to one of claims 4 to 7, **characterized in that** the proximal shaft portion (2) has an eccentric locking slug (12), which is fixedly arranged inside an outer tube (6) of the proximal shaft portion (2) and in which the bushing (14) is rotable and supported decentrally with respect to a central axis of the proximal shaft portion (2).

9. The shaft (1) according to one of claims 1 to 8, **characterized in that** the transmission component/element (18) is firmly connected at a distal end (28) to an adjustment bushing (16), which transmits the rotary movement of the transmission component/element (18) to the distal shaft portion (4).

10. The shaft (1) according to claim 9, **characterized in that** the transmission component/element (18) is welded to an inner side of the adjustment bushing (16).

11. The shaft (1) according to one of claims 1 to 10, **characterized in that** the transmission component/element (18) is installed in the straight construction position of the two shaft portions (2, 4) at an angle of 22.5°, such that that the proximal end portion (26) of the transmission component/element (18) is aligned parallel to the center axis of the proximal shaft portion (2) and the distal end portion (28) of the transmission component/element (18) is aligned at the angle of 22.5° with respect to the center axis of the distal shaft portion (4).

12. The shaft (1) according to one of claims 1 to 11, **characterized by** an outer diameter of less than 5.6 mm.

13. The shaft (1) according to one of claims 1 to 12, **characterized in that** the transmission component/element (18) is in one piece.

14. A medical hand instrument (42) comprising a shaft (1) according to one of claims 1 to 13 and a proximal handpiece (44).

## Revendications

1. Arbre (1) d'un ou pour un instrument médical manuel (42), de préférence d'un instrument chirurgical de conception à invasion minimale, avec une section d'arbre proximale (2) et une section d'arbre distale (4) qui se tord par rapport à la section d'arbre proximale (2) tout en formant un angle, et
un train d'engrenage (7), qui est disposé à l'intérieur de l'arbre (1) et transmet un mouvement de rotation du train d'engrenage (7) dans la section d'arbre proximale (2) sur la section d'arbre distale (4),
dans lequel le train d'engrenage (7) présente une pièce/un élément de transmission (18) tubulaire qui transmet le mouvement de rotation à la section d'arbre distale (4) et permet la formation d'un angle entre les deux sections d'arbre (2, 4),
dans lequel la pièce/l'élément de transmission (18) présente ou est une pièce tubulaire avec deux sections d'extrémité (26, 28) espacées axialement et une section de flexion centrale (31), dans laquelle une pluralité d'évidements (32) s'étendant dans une direction périphérique, ainsi que espacés dans une direction longitudinale tubulaire, sensiblement de formes ovale et elliptique, ainsi que dans une direction périphérique, sont formés avec des extrémités d'évidement se terminant en pointe qui sont disposés les uns par rapport aux autres de telle sorte qu'il en résulte un modèle d'évidement diagonal dans le cas d'un déroulement de la section de flexion centrale (31) dans le plan.

2. Arbre (1) selon la revendication 1, **caractérisé en ce que** les évidements (32) espacés axialement se chevauchent dans une direction périphérique de la pièce/de l'élément de transmission (18) dans des sections de chevauchement (38) et une longueur des sections de chevauchement (38) représente de préférence respectivement un tiers de la longueur des évidements (32).

3. Arbre (1) selon la revendication 2, **caractérisé en ce que** les sections de chevauchement (38) excluent une zone de la plus grande épaisseur des évidements (32).

4. Arbre (1) selon l'une quelconque des revendications 1 à 3, **caractérisé en ce que** la pièce/l'élément de transmission (18) est relié(e) de manière fixe en rotation avec une douille (14) au niveau d'une section d'extrémité proximale (26) qui transmet le mouvement de rotation du train d'engrenage (7) à l'intérieur de la section d'arbre proximale (2) à l'élément de transmission (18).

5. Arbre (1) selon la revendication 4, **caractérisé en ce que** la pièce/l'élément de transmission (18) présente au niveau de la section d'extrémité proximale (26) plusieurs fentes longitudinales (34) qui s'ouvrent au niveau de la section d'extrémité proximale (26) et sont prévues et formées pour recevoir respectivement un nez de mise en prise (36) faisant saillie radialement à partir de la douille (14).

6. Arbre (1) selon la revendication 4 ou 5, **caractérisé en ce que**le train d'engrenage (7) présente la douille (14), une couronne (8) et un pignon (10) qui est entraîné par la couronne (8), et la douille (14) est reliée fixement au pignon (10).

7. Arbre (1) selon la revendication 6, **caractérisé en ce que** la douille (14) est reliée au pignon (10) par un ajustement par serrage.

8. Arbre (1) selon l'une quelconque des revendications 4 à 7, **caractérisé en ce que** la section d'arbre proximale (2) présente une douille de sécurisation excentrée (12) qui est disposée fixement à l'intérieur d'un tube extérieur (6) de la section d'arbre proximale (2) et dans lequel la douille (14) est montée de manière rotative ainsi que de manière décentralisée par rapport à un axe central de la section d'arbre proximale (2).

9. Arbre (1) selon l'une quelconque des revendications 1 à 8, **caractérisé en ce que** la pièce/l'élément de transmission (18) est relié(e) fixement avec une douille de réglage (16) au niveau d'une extrémité distale (28), douille qui transmet le mouvement de rotation de la pièce/de l'élément de transmission (18) à la section d'arbre distale (4).

10. Arbre (1) selon la revendication 9, **caractérisé en ce que** la pièce/l'élément de transmission (18) est soudé(e) à un côté intérieur de la douille de réglage (16).

11. Arbre (1) selon l'une quelconque des revendications 1 à 10, **caractérisé en ce que** la pièce/l'élément de transmission (18) est intégré(e) de manière à être plié selon un angle de 22,5 dans une position de construction droite des deux sections d'arbre (2, 4), de telle manière que la section d'extrémité proximale (26) de la pièce/de l'élément de transmission (18) est orientée parallèlement à l'axe central de la section d'arbre proximale (2) et de la section d'extrémité distale (28) de la pièce/de l'élément de transmission (18) selon un angle de 22,5 ° par rapport à l'axe central de la section d'arbre distale (4).

12. Arbre (1) selon l'une quelconque des revendications 1 à 11, **caractérisé par** un diamètre extérieur inférieur à 5,6 mm.

13. Arbre (1) selon l'une quelconque des revendications 1 à 12, **caractérisé en ce que** la pièce/l'élément de transmission (18) est réalisé(e) d'une seule pièce.

14. Instrument médical manuel (42) avec un arbre (1) selon l'une quelconque des revendications 1 à 13 et une poignée proximale (44).
